# EUROPÄISCHE PATENTSCHRIFT

(11) **EP 1 311 304 B1**
(45) Veröffentlichungstag und Bekanntmachung des Hinweises auf die Patenterteilung: **29.09.2004**
(21) Anmeldenummer: 01971810.5
(22) Anmeldetag: 28.07.2001
(51) Int. Cl.: A61L 2/20

(54) **VORRICHTUNG ZUM STERILISIEREN VON PACKUNGEN MIT WASSERSTOFFPEROXID**
DEVICE FOR STERILISING PACKAGING USING HYDROGEN PEROXIDE
DISPOSITIF DE STERILISATION D'EMBALLAGES AU PEROXYDE D'HYDROGENE

(30) Priorität: 21.08.2000 DE 10040861
(43) Veröffentlichungstag der Anmeldung: 21.05.2003
(73) Patentinhaber: Tetra Laval Holdings & Finance S.A., 1009 Pully (CH)
(72) Erfinder: ARMBRUSTER, Hans, 68623 Lampertheim (DE); RÖMER, Michael, 65428 Rüsselsheim (DE); SÖRENSEN, Karsten, B., 64347 Griesheim (DE); WOLF, Michael, 64283 Darmstadt (DE)
(74) Vertreter: Weber, Dieter, Dr.
(86) Internationale Anmeldenummer: PCT/EP2001/008779
(87) Internationale Veröffentlichungsnummer: WO 2002/015946

(56) Entgegenhaltungen:
- EP-A- 0 758 611
- US-A- 4 742 667

## Beschreibung

Die Erfindung betrifft eine Vorrichtung zum gleichzeitigen Sterilisieren einer Mehrzahl von Packungen mit Hilfe eines Wasserstoffperoxid und ein Trägergas enthaltenden Gasgemisches,
- mit Zuführleitungen für das Trägergas und für Wasserstoffperoxid,
- einer Einrichtung zum Verdampfen von Wasserstoffperoxid mit Wärme und zum Einmischen in das Trägergas,
- mit Zuführleitungen und einer im wesentlichen horizontal verlaufenden Verteilerleitung und
- mit über der jeweiligen Packung angeordneten und mit der Verteilerleitung verbundenen Düsen.

Aus der EP 0 758 611 A ist bekannt, oben offene Packungen durch Einleiten eines Gemisches aus Luft und gasförmigem Wasserstoffperoxid zu sterilisieren. Bei diesem bekannten Verfahren werden mehrere, in einer Reihe nebeneinander angeordnete Packungen gleichzeitig unter die Öffnungen entsprechend vieler Düsen geführt. Das Sterilisierungsgasgemisch wird dadurch hergestellt, daß Heißluft durch ein poröses Rohrstück geführt wird, durch welches von außen vollständig verdampftes Wasserstoffperoxid gedrückt und dann mit der Luft vermischt wird. Femer wird zur Vermeidung einer Kondensation des Sterilisierungsmittels das Gemisch durch eine Zuführ- und eine Verteilerleitung geführt, die doppelwandig ausgestaltet und mit heißem Dampf beschickt sind.

Die Ausgestaltung der Leitungen mit beheizten Rohrwänden ist kostenaufwendig, schwierig und umständlich zu fertigen, und das Eindiffundieren des verdampften Wasserstoffperoxides in dem versinterten Rohrteil in die Heißluft ist ebenfalls mit Problemen verbunden, weil man sehr hohe Pumpdrücke für das Wasserstoffperoxid benötigt und man Vorsorge dafür treffen muß, daß Poren mit der richtigen Größe nicht blockieren. Ein Zusetzen der Poren kann bei kleinen Porengrößen erwartet werden, während bei zu großen Poren das Wasserstoffperoxid nur mangelhaft verdampft wird.

Ferner ist es aus der US-4,742,667 bekannt, Verpackungen durch verdampftes Wasserstoffperoxid zu sterilisieren, welches mittels Druckluft in eine Packung geblasen wird. Diese bekannte Sterilisierungsvorrichtung eignet sich nur für eine Reihe von Packungen, da sie aus einer Bedampfungsdüse und mehreren nachgeschalteten Trocknungsdüsen besteht. In nachteiliger Weise bedeutet es einen hohen materiellen und technischen Aufwand, für jede Reihe einen Verdampfer und eine Düse zum Dosieren des Peroxids vorzusehen. Die Folge wären Störanfälligkeit und ein ungleichmäßiger Sterilisationsprozeß, sowohl quer über die Reihen betrachtet als auch im Takt der nachfolgenden Behälter. Weiterhin gäbe es einen hohen Verbrauch an Sterilisationsmittel, weil jede einzelne Sterilisierung mit einer Überdosis an Sterilisationsmittel arbeiten muß, um den Prozeß sicher zu gewährleisten.

Der Erfindung liegt daher die Aufgabe zugrunde, die Vorrichtung der eingangs genannten Art zu vereinfachen und gleichzeitig Maßnahmen vorzusehen, um die Konzentration des Wasserstoffperoxids an allen Düsen über der Vielzahl von Packungen zu bestimmter Zeit gleich groß zu halten.

Die Lösung dieser Aufgabe gelingt gemäß der Erfindung dadurch, daß die Verteilerleitung von der aufstromigen Stelle der Einspeisung des konditionierten Gasgemisches bis zur Stelle des Eintrittes vor die jeweilige Düse als einen Längskanal enthaltender Rohrkörper ausgestaltet ist mit mindestens einer sich über dessen Länge erstreckender, etwa rohrförmigen Heizpatrone und verteilten Meßstellen, und daß die Heizpatrone in wenigstens zwei Abschnitte aufgeteilt und durch Zufuhr elektrischer Energie gesteuert derart erwärmbar ist, daß sich die Temperatur an den äußeren Enden des Rohrkörpers von der in der Mitte unterscheidet und daß vorzugsweise die Temperatur an den Enden von der Temperatur in der Mitte unabhängig eingestellt werden kann.

Durch die Erfindung ist es im Vergleich zum Stand der Technik nicht mehr erforderlich, die Verteilerleitung oder gar auch die Zuführleitung doppelwandig zum Beheizen der Rohrleitung mit Hilfe des Durchleitens von heißem Dampf auszugestalten. Es genügt der Einsatz eines Längskanales mit in dessen Längsrichtung angeordneter Heizpatrone, um in dem Längskanal ein bestimmtes Temperaturprofil aufzubauen. Es ist verständlicherweise einfacher, einen einwandigen Längskanal auszubilden oder diesen in einem Rohrkörper vorzusehen und gleichzeitig Heizpatronen anzuordnen. Man hat festgestellt, daß es außerdem günstiger ist, das Gasgemisch nach dessen Herstellung und Einleitung in die Verteilerleitung in letzterer durch Heizpatronen zu erwärmen, zumal entsprechend der Aufteilung der Heizpatrone in mindestens zwei Abschnitte ein Temperaturprofil in gewünschter Größe recht einfach aufzubauen ist. Man hat überraschend festgestellt, daß die Temperatur des Gasgemisches an der Verteilerleitung und insbesondere an deren Enden eine Veränderung der Konzentration des Peroxides in der Gasphase bewirkt. Daher ist es erfindungsgemäß besonders bevorzugt, die Temperatur an den äußeren Enden des Rohrkörpers niedriger zu halten als in dessen Mitte. Durch die entsprechende Erwärmung der Heizpatrone in der Mitte des Rohrkörpers kann man einer Erniedrigung, d.h. einer Abkühlung des Gasgemisches an den notwendigen Stellen entgegenwirken mit der Folge, daß die Temperatur an den äußeren Endbereichen des Rohrkörpers niedriger ist als an dessen Mittelbereich, wodurch überraschenderweise sowohl eine überall gleichbleibende Temperatur des aus dem Rohrkörper ausströmenden Gasgemisches als auch eine überall gleichbleibenden Konzentration des darin enthaltenen H₂O₂ erzielt wird.

Bei der erfindungsgemäßen Sterilisierungsvorrichtung kann man 5, 10 oder 20 Packungen gleichzeitig sterilisieren, d.h. mit dem Sterilisierungsgas durch die Düsen beaufschlagen. Die zu sterilisierenden Packungen werden wie im bekannten Fall in einer Reihe, die vorzugsweise in einer geraden Linie liegt, angeordnet. Der die Verteilerleitung darstellende Rohrkörper erstreckt sich dann über diese Reihe der Packungen, hat also die Form einer länglichen Leiste oder eines länglichen Kanales. Bevorzugt wird das erzeugte Gasgemisch in der Mitte der Längserstreckung des Rohrkörpers eingeleitet und dieser so beheizt, daß sich die Temperatur des Gases zu den äußeren Enden des Rohrkörpers hin verringert. Die Erfindung besorgt eine Erwärmung der Wandungen des Längskanales im Mittelbereich und gegebenenfalls auch des gesamten Rohrkörpers im Verhältnis zu dessen äußeren Enden, obwohl die Einspeisestelle des Gasgemisches im Mittelbereich liegt. Dadurch wird erfindungsgemäß eine ungleichmäßige Erwärmung und Verteilung der Konzentration des Peroxids in dem Gasgemisch vermieden.

Bei einem konkreten, bevorzugten Ausführungsbeispiel hat man Packungen mit einem Fassungsvermögen von 0,5 Liter, 1 Liter und 1,5 Liter jeweils zu zehnt gleichzeitig sterilisiert. Der gesamte Volumenstrom an Luft für jeweils 10 Flaschen betrug 20 - 30 Nm³/h, und besonders bevorzugt 22 Nm³/h. Die Temperatur der Luft sollte im Bereich zwischen 110° und 150°C liegen und besonders bevorzugt 140°C betragen. Es wurde 25%iges Wasserstoffperoxid flüssig pro Takt, d.h. pro 10 Flaschen, zudosiert, und zwar für die 0,5 Liter Packung 0,5 bis 1,0 ml, bevorzugt 0,75 ml; für eine 1 Liter Packung: 1,0 bis 1,5 ml, bevorzugt 1,0 ml; und für eine 1,5 Liter Packung: 1,75 bis 2,5 ml und besonders bevorzugt 2,0 ml. Als Volumenstrom für flüssiges Wasserstoffperoxid sollte eine Größe von 1,5 bis 1,9 ml/s und besonders bevorzugt 1,7 ml/s verwendet werden. Die Gaskonzentration lag im Bereich von 50 bis 93 g H₂O₂ /Nm³ Luft und besonders bei einem günstigen Beispiel bei 77 g H₂O₂/Nm³ Luft.

Bei vorteilhafter Ausgestaltung der Erfindung hat der Rohrkörper die Form eines länglichen Steges mit einem massiven Grundkörper, der mittig von einer den Gaskanal bildenden Längsbohrung mit Verbindungsöffnungen zu den Düsen durchzogen ist und der auf gegenüberliegenden Seiten des Gaskanales und im Abstand zu diesem mit nach außen offenen Nuten für das Einlegen einer Heizpatrone versehen ist. Die Herstellung der Verteilerleitung ist mit diesen Maßnahmen besonders einfach, denn aus einem stegartigen, massiven Grundkörper kann man einen derart aufgebauten Rohrkörper leicht fertigen. Es braucht nur mittig eine Längsbohrung in Längsrichtung des Steges vorgesehen werden, die entsprechend der Anzahl der beabsichtigten Düsen mit Verbindungsöffnungen quer zur Längsrichtung des Steges versehen wird. Im Bereich dieser Verbindungsöffnungen werden dann die Düsen angebracht, zum Beispiel "unten" an dem Rohrkörper und Grundkörper, weil sich die Sterilisierungsgase und auch andere Behandlungsmittel in nachgeschalteten Behandlungsstationen der Gesamtmaschine zweckmäßig von oben nach unten einleiten bzw. nach Beaufschlagung der Oberflächen der Innenwände der Packungen nach oben wieder absaugen lassen. Deshalb wird der Rohrkörper oberhalb der Düsen und werden diese oberhalb der zu behandelnden Packungen angeordnet.

Während die Verbindungsöffnungen zu den Düsen dann im Boden des Grundkörpers vorgesehen werden, ordnet man erfindungsgemäß ferner auf gegenüberliegenden Seiten, also seitlich des Rohr- bzw. Grundkörpers jeweils eine nach außen offene Nut an, weil eine oder mehrere Heizpatronen besonders einfach dann neben die Längsbohrung anzuordnen ist/sind. Nach außen sorgen Klemmund Druckstücke für ein Verschließen und Isolieren gegen abströmende Wärme.

Besonders günstig ist es erfindungsgemäß, wenn jeder Abschnitt der Heizpatrone unabhängig von dem anderen beheizbar ist. Durch eine solche unabhängige Regelung einzelner oder jedes Heizpatronenabschnittes im Verhältnis zu dem benachbarten Abschnitt kann man das gewünschte Temperaturprofil einstellen mit der Folge, daß zum Beispiel die Temperatur in dem Rohrkörper von der Mitte nach außen hin abfällt. Mißt man bei einer Vorrichtung, welche diese erfindungsgemäßen Merkmale beinhaltet, an dem jeweiligen Düsenende die Temperatur, dann ist eine Regelung so möglich, daß die Temperatur des Gases am Düsenende im Zeitpunkt des Ausströmens für das Sterilisieren bei allen Packungen der gerade behandelten Reihe gleich ist. Damit hat man in allen Pakkungen die gleiche Wasserstoffperoxidkonzentration mit der Folge der gleichen Sterilisierung.

Zweckmäßig ist es gemäß der Erfindung ferner, wenn die jeweilige Düse, vorzugsweise über eine Düsenhalteplatte, mit den Verbindungsöffnungen des Rohrkörpers verbunden und an letzterem angebracht ist und eine Drosselöffnung hat. Diese Drosselöffnung kann unterschiedlich gebildet werden. Sie befindet sich zum Beispiel an der aufstromigen Eingangsseite der Düse, während sich die sogenannten Sprühlöcher am abstromseitigen Ende der Düse befinden; bei einer normalen länglichen Düse ein einziges Sprühloch. Bei der zuletzt erwähnten Ausführungsform der länglichen Düse kann die Drosselöffnung sogar identisch mit dem Sprühloch sein und sich am Düsenende befinden. Bei einer anderen alternativen Ausführungsform kann sich hingegen die Drosselöffnung aufstromseitig und im Abstand vom Düsenende befinden. In jeder Ausführungsform sollte jedoch die jeweilige Düse mit einer Drosselöffnung versehen sein, um einen Rückstau des Gasgemisches zu bewirken mit der Folge, daß die Drosselung und damit der Druckverlust über die Länge des Gaskanales hinweg vernachlässigbar klein sind. Hingegen soll die Drosselöffnung der Düse einen wesentlich größeren Druckverlust bewirken. Dadurch wird auch der Volumenstrom des aus jeder Düse austretenden Gasgemisches beeinflußt und ist bei Beachtung der erfindungsgemäßen Lehre weitgehend gleich.

Mit der erfindungsgemäßen Vorrichtung kann man sowohl oben offene Packungen im Inneren als auch oben geschlossene Packungen an ihrer äußeren Oberfläche sterilisieren. Im ersteren Fall kann sich die Drosselöffnung am Düsenende unten befinden, während die aufstromig davor liegenden Leitungen und Öffnungen eine wesentlich geringere Drosselwirkung haben, weil die Durchtrittsöffnungen größer sind.

Bei der zweitgenannten Ausführungsform, bei welcher zum Beispiel eine als Flasche ausgestaltete Packung in ihrem oberen Gewindehalsbereich noch verschlossen ist und von außen sterilisiert wird, wird die Gleichheit der aus den einzelnen Düsen austretenden Volumenströme durch eine Drosselöffnung in einer Blende erreicht, die im Bereich der Eintrittsstelle des konditionierten Gasgemischen zwischen Verteilerleitung und Düse angeordnet ist. Diese Eintrittsstelle kann sich vorzugsweise im Bereich der jeweiligen Verbindungsöffnung zu den Düsen befinden.

Bei derjenigen Ausführungsform der erfindungsgemäßen Sterilisierungsvorrichtung, bei welcher oben offene Packungen in ihrem Innenraum sterilisiert werden, ist die jeweilige Düse der in einer Reihe unter dem Rohrkörper befestigt angebrachten Düsen länglich ausgestaltet mit einem Befestigungsflansch oben für die Anbringung an einer Düsenhalteplatte und mit einem weiteren länglichen Durchströmkanal, der sich etwa über zwei Drittel der Düsenlänge erstreckt, sowie im Bereich des Düsenendes mit einem engeren Austrittskanal, welcher zugleich die Funktion der Drosselöffnung übernimmt. Bei dieser Ausführungsform wird die Kante der jeweils oben offenen Packung vertikal im Abstand unter dem unteren Düsenende gehalten. Dadurch können die Packungen horizontal unter den Düsen vorbei transportiert werden. Am äußeren Umfang des unteren Düsenendes können auch ringförmige Saugeinrichtungen vorgesehen sein, um die aus dem Inneren der mit sterilisierendem Gas beaufschlagten Packungen austretenden Abgase aufzunehmen, abzusaugen und abzuführen.

Es gibt auch flaschenförmig ausgestaltete Packungen, die oben eine mit einem Außengewinde versehene Öffnung am Flaschenhals haben. Bei der Herstellung einer solchen Flasche, die meist aus HDPE besteht und mit Extrusions-Blasverfahren gefertigt wird, ist die Flasche nach der Herstellung inwendig steril und die Öffnung am Flaschenhals oben in diesem Zwischenstadium noch durch einen Dom verschlossen, der bei einer späteren Bearbeitungsstation abgeschnitten wird. Im Bereich des Schnittes darf keine Verunreinigung in das Innere einer solchen Packung kommen. Deshalb ist es bevorzugt, im Bereich des Flaschenhalses und des Domes darüber bei noch verschlossener Packung die äußeren Oberflächen zu sterilisieren. Dementsprechend weist erfindungsgemäß die Düse einen die zu sterilisierenden Bereiche der Packung wenigstens teilweise umgreifenden, durch einen auf gegenüberliegenden Seiten offenen, rinnenförmigen, mit Sprühlöchern versehenen Sprühkanal begrenzten Innenraum und im Abstand vom Sprühkanal angebrachte, ebenfalls seitlich teilweise offene Außenwände auf. Es wurde oben bereits erwähnt, daß bei dieser Ausführungsform die Drosselöffnung der Düse oben an der Eintrittsstelle des konditionierten Gasgemisches im Bereich der Verbindungsöffnung liegt. Im Abstand von dieser Drosselöffnung oder Blende befindet sich unterhalb und innerhalb von Außenwänden sowie im Abstand von diesen der erwähnte rinnenförmige Sprühkanal. Dieser ist in demjenigen Bereich, in welchem die zu sterilisierende Packung für die Behandlung zum Stillstand kommt, mit Sprühlöchern versehen. Mit anderen Worten befinden sich die Sprühlöcher zwar wenigstens teilweise auch in der Rinne oben, vorzugsweise und hauptsächlich aber auf gegenüberliegenden Seiten der Rinnenwände. Auf den anderen beiden Seiten ist die Rinne auf gegenüberliegenden Seiten offen, weshalb es sich um einen Sprühkanal in Rinnenform handelt. In diese Rinne taucht nämlich der obere geschlossene Teil der Packung ein und wird längs der Rinne hindurchgeführt, im Bereich der Sprühlöcher intermittierend angehalten und danach weitergeführt, je nachdem wann die Reihe von Packungen an die Düse herangeführt und nach Behandlung weitergefördert wird.

Für die Behandlung dieser Ausführungsform der oben geschlossenen Packungen, vorzugsweise der oben am Flaschenhals mit einem Dom noch verschlossenen Flaschen, taucht der obere Bereich dieser Packung in den rinnenförmigen Sprühkanal ein. Durch diesen Sprühkanal wird der Innenraum begrenzt, in welchen das Oberteil der Packung eintaucht, wobei der Innenraum deshalb den oberen Teil der Packung umgreift. Der obere Teil der Packung soll äußerlich sterilisiert werden. Im Abstand von den Rinnenwänden des Sprühkanals nach außen quer zur Längsrichtung der Rinne sind die Außenwände der Düse angebracht, so daß sich jeweils nach außen hinter den Sprühlöchern ein Raum bildet, durch welchen das konditionierte Gasgemisch von dem aufstromseitigen Bereich der Düse durch die Sprühlöcher zu der zu sterilisierenden Oberfläche der Packung geführt werden kann.

Nach dem Aufsprühen strömt das verbrauchte Gasgemisch einerseits an der unteren Kante des Sprühkanals nach außen ab, vorzugsweise oberhalb einer Flaschenhalteplatte, wenn eine solche vorgesehen ist, und strömt auch längs des Sprühkanals nach außen ab. Dort kann der entweder nicht verwendete oder verbrauchte Anteil des Gasgemisches abgezogen und zur Wiederaufbereitung gesammelt werden.

Es ist erfindungsgemäß weiterhin zweckmäßig, wenn die Einrichtung zum Verdampfen von Wasserstoffperoxid mit Wärme und zum Einmischen in das Trägergas eine von einer Zuführleitung für flüssiges Wasserstoffperoxid gespeiste Vernebelungsdüse aufweist, die im mittleren Bereich einer Verdampfungskammer aufstromig eines Erwärmungskörpers angeordnet ist, zu dessen aufstromiger, warmer Oberfläche Wasserstoffperoxid in Nebelform unter Vermischen mit warmem Trägergas zugeführt und durch Kanäle in den Erwärmungskörper derart geleitet wird, daß vor der Einspeisung in die Verteilerleitung eine Überhitzung des Gasgemisches erfolgt. Zum Erzeugen und Konditionieren des sterilisierenden Gasgemisches wird also gasförmiges Wasserstoffperoxid mit Trägergas, vorzugsweise heißer, steriler Luft, vermischt und dann der jeweiligen Düse zugeführt. Dieses Herstellen und Konditionieren des Gasgemisches erfolgt zunächst zentral in der erwähnten Verdampfungs- und Einmischeinrichtung. Diese ist bei der hier diskutierten bevorzugten Ausführungsform ein Erwärmungskörper mit vorgeschalteter Verdampfungskammer. Heißluft wird über Pumpen der Verdampfungskammer zugeführt, die zum Beispiel wie ein umgekehrter Trichter auf einer ebenen, warmen Oberfläche des Erwärmungskörpers angebracht sein kann. Etwa in der Mitte dieser Verdampfungskammer befindet sich das abstromseitige Ende einer Vernebelungsdüse, welcher flüssiges Wasserstoffperoxid zugeführt wird. Dieser H₂O₂-Nebel wird zunächst mit der Heißluft vermischt, gelangt dann auf die erwähnte ebene, warme Oberfläche, verdampft und wird schließlich in den Kanälen in dem Erwärmungskörper erhitzt. Danach ist das Gasgemisch konditioniert und kann zentral dem Rohrkörper und danach entlang diesem den einzelnen Düsen zugeführt werden.

Bei einem solchen Konditionierverfahren erfolgt zunächst das Aufsprühen des Sterilisierungsmittels auf die erwähnte warme Oberfläche, die sich auf einer ersten Temperatur befindet. Diese Oberfläche ist kleiner als diejenige, bei welcher ein Filmsieden beginnt. Das Gasgemisch setzt sich nämlich in Form eines Filmes ab und beginnt dann auf diesen Oberflächen zu sieden. Der Erwärmungskörper gestattet ein Überhitzen des Sterilisierungsmittels, welches von der erwähnten warmen Oberfläche längs der Kanäle in dem Erwärmungskörper nach unten geleitet wird. Im Gegenstrom dazu, also von unten nach oben, kann ein Wärmefluß in dem Erwärmungskörper erzeugt werden, welcher die Überhitzung besorgt.

Dazu ist es zweckmäßig, wenn erfindungsgemäß im abstromigen Endbereich des Erwärmungskörpers einige Heizstäbe eingesteckt sind.

Bei der erfindungsgemäßen Sterilisierungsvorrichtung wird das Sterilisierungsmittel - wie vorstehend erwähnt - durch Aufsprühen in Nebelform mit der oberen warmen Oberfläche des Erwärmungskörpers in Berührung gebracht. Es kommt nicht eine Flüssigkeit (das Sterilisierungsgemisch) mit der oberen warmen Oberfläche in Berührung, sondern nur ein Nebel des Sterilisierungsmittels, also eine große Anzahl fein verteilter Tröpfchen. Dadurch werden die Wirkoberfläche des flüssigen Sterilisierungsmittels erheblich vergrößert und der Wärmeübergang von der warmen Oberfläche auf das jeweilige Flüssigkeitströpfchen verbessert.

Damit eine Vernebelung am Ende der Vernebelungsdüse erfolgreich durchgeführt werden kann, ist bei weiterer Ausgestaltung der Erfindung die Zuführleitung für flüssiges Wasserstoffperoxid durch ein Kühlfließmittel gekühlt. Vorzugsweise kann man die Vernebelungsdüse, die länglich ausgestaltet ist und von ihrer Zuführstelle bis in die Mitte der Verdampfungskammer reicht, außen mit einem Mantel versehen, der von Kühlmittel durchflossen ist, zum Beispiel Wasser. Die technische Herstellung einer solchen außen gekühlten, länglichen Düse ist ohne Probleme einwandfrei beherrschbar.

Das Einstecken von rohrförmigen Heizstäben, zum Beispiel von unten in den abstromigen Endbereich des Erwärmungskörpers, ist ebenfalls technisch problemlos. Man kann dadurch einen Wärmegradienten von unten nach oben und damit einen Wärmefluß in dem Erwärmungskörper erreichen, der der Strömung des in Verdampfung befindlichen Gasgemisches entgegenläuft. Auf diese Weise ist unter sparsamem Energieeinsatz eine effektive Überhitzung des Sterilisierungsgases möglich. Dabei sollte stets die erfindungsgemäße Empfehlung beachtet werden, daß das abstromseitige Ende der Vernebelungsdüse sich im Abstand von den Wandungen der Verdampfungskammer befindet, der Wasserstoffperoxidnebel also nicht auf eine Wandung, sondern frei in den Raum der Verdampfungskammer gesprüht wird.

Selbst wenn PET- und HDPE-Verpackungen mit Hilfe von Wasserstoffperoxid sterilisiert werden, sind die bestehenden Hygiene- und Gesundheitsbedingungen erfüllt. Es ist möglich, die H₂O₂-Restmenge in mit Flüssigkeit gefüllten PET-Flaschen unter 0,5 ppm zu halten.

Bei der Herstellung, Sterilisierung, Abfüllung und dem Verschließen von Packungen unter Zuhilfenahme der erfindungsgemäßen Vorrichtung hat sich bei einer besonders bevorzugten leistungsstarken Maschine eine Taktzeit von etwa 5,7 Sekunden, einschließlich der Transportzeit von Station zu Station ergeben. Die jeweilige Packung verweilt also etwa 4,5 bis 5,5 Sekunden unter der Düse, bei einer speziellen Ausführungsform betrug diese Zeit 4,7 Sekunden.

Man erreichte an den Enden von zehn in einer Reihe hintereinander angeordneten Düsen Temperaturen, die weniger als 10°C voneinander abwichen mit Höchsttemperaturen von 143°C und Tiefsttemperaturen von 134°C bei einer bevorzugten Ausführungsform.

Weitere Vorteile, Merkmale und Anwendungsmöglichkeiten der vorliegenden Erfindung ergeben sich aus der folgenden Beschreibung in Verbindung mit den anliegenden Zeichnungen. Bei diesen zeigen:
- Figur 1: eine schematische Übersicht einer Sterilisierungsvorrichtung, bei welcher insbesondere die Verteilerleitung mit den nach unten wegstehenden Düsen und in der Mitte nach oben ragend der Erwärmungskörper dargestellt sind,
- Figur 2: eine isometrische Darstellung des länglichen Rohrkörpers mit dem mittig aufragend angebrachten Erwärmungskörper, der darüber angeordneten Verdampfungskammer und den Zuführleitungen,
- Figur 3: einen vertikalen Längsschnitt durch die Vorrichtung der Figur 2,
- Figur 4: einen abgebrochenen horizontalen Schnitt der rechten Hälfte des Rohrkörpers,
- Figur 5: einen Längsquerschnitt durch eine Heizpatrone mit drei Zonen,
- Figur 6: eine Querschnittsansicht des Rohrkörpers, wobei die Schnittebene senkrecht auf der Papierebene der Figur 3 steht,
- Figur 7: eine Düse einer besonderen Ausführungsform mit rinnenförmigem Sprühkanal zum Aufblasen von Sterilisierungsgas auf die äußere Oberfläche des Oberteils einer Packung,
- Figur 8: eine abgebrochene Querschnittsansicht durch die Düse einer anderen Ausführungsform für das Einblasen von konditioniertem Gas in das Innere einer Packung durch deren obere Öffnung und
- Figur 9: eine teilweise aufgebrochene Seiten- und Querschnittsansicht einer Vernebelungsdüse.

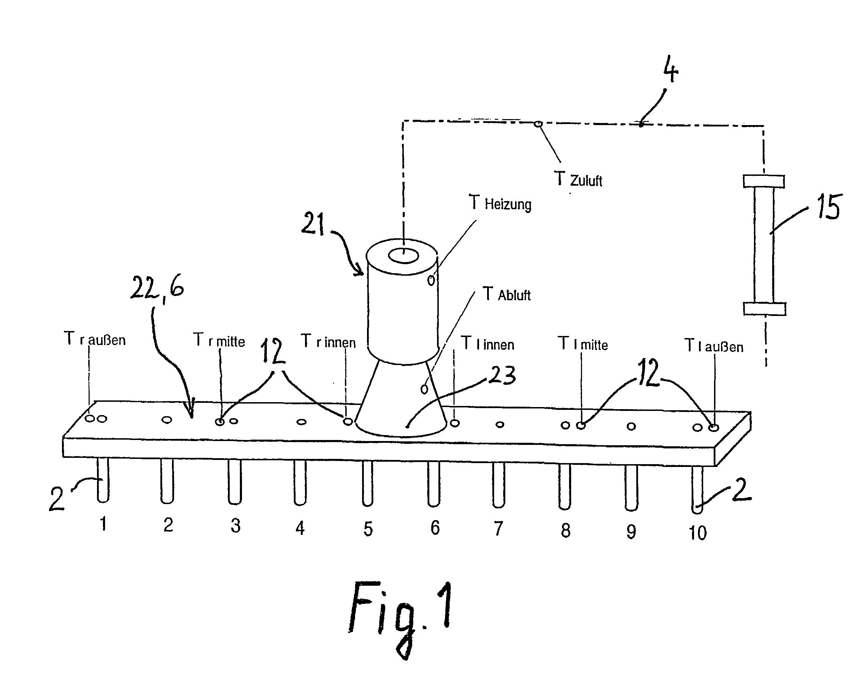

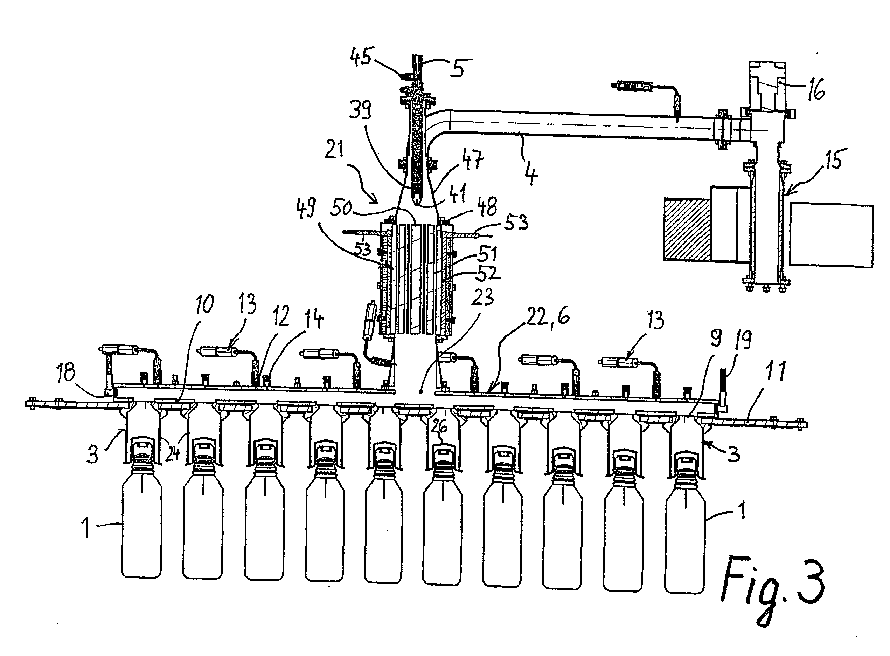

Zum gleichzeitigen Sterilisieren sind bei den in den Zeichnungen dargestellten bevorzugten Ausführungsformen zehn Packungen 1 in einer Reihe hintereinander gemäß Darstellung der Figur 3 unter einer Düse 2 bzw. 3 angeordnet dargestellt. Über Zuführleitungen 4, 5 werden sterile Warmluft als Trägergas einerseits und flüssiges Wasserstoffperoxid andererseits einer Einrichtung 21 zum Verdampfen des Wasserstoffperoxid mit Wärme und zum Einmischen in die Warmluft geleitet, so daß das konditionierte Gasgemisch schließlich in die allgemein mit 22 bezeichnete Verteilerleitung und von dort in die Düsen 2, 3 geführt werden kann. Von der aufstromigen Stelle 23 für die Einspeisung des konditionierten Gasgemisches durchstreicht dieses die Verteilerleitung 22 von der in der Mitte der Verteilerleitung 22 angeordneten Stelle 23 in der linken Hälfte gemäß den Figuren 1 bis 3 nach links und in der rechten Seite nach rechts bis zu dem linken bzw. rechten äußeren Ende der Verteilerleitung 22.

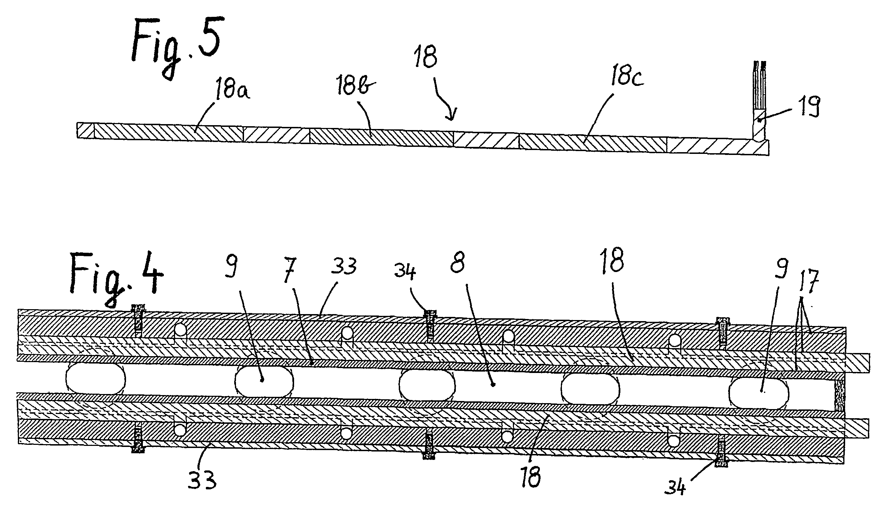

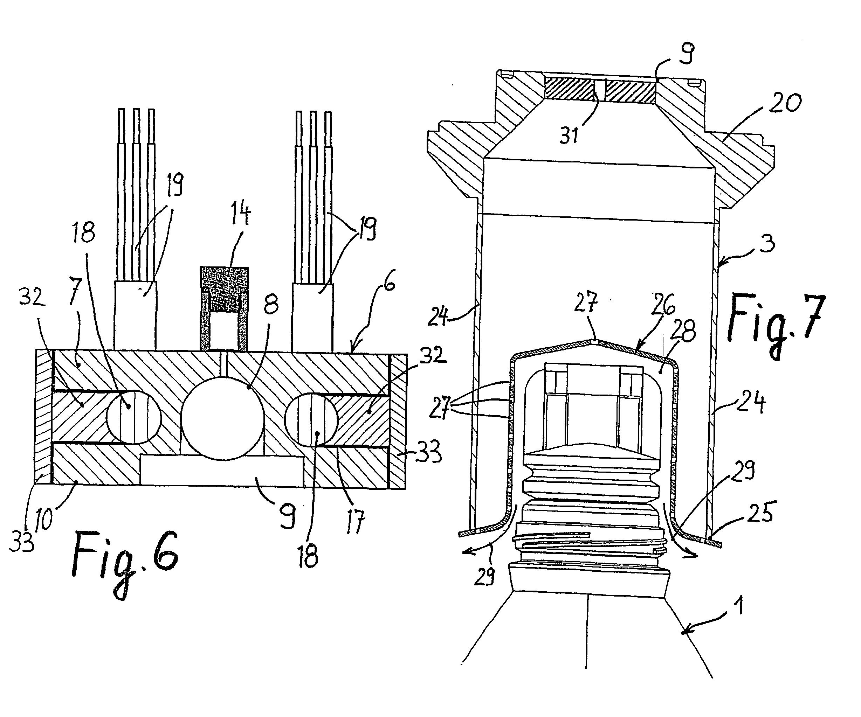

Die Verteilerleitung 22 ist als Rohrkörper 6 in Form eines länglichen Steges ausgestaltet. Dieser besteht gemäß Darstellung der Figuren 4 und 6 aus einem massiven Grundkörper 7, der mittig von einem als Gaskanal ausgebildeten Längskanal 8 durchzogen ist. Diesen kann man dadurch herstellen, daß man den Grundkörper 7 in Längsrichtung durchbohrt. Deshalb ist der Längskanal 8 als Gaskanal in Figur 6 mit kreisrundem Querschnitt gezeigt. In Figur 4 blickt man auf die untere Halbrinne dieses Längskanals 8 und sieht im Abstand voneinander angeordnet fünf Verbindungsöffnungen 9, welche eine direkte Verbindung zu den an diesen Verbindungsöffnungen 9 angebrachten Düsen 2 bzw. 3 vorsieht. Deckungsgleich mit diesen Verbindungsöffnungen 9 kann unter dem Boden 10 des Rohrkörpers 6 eine Düsenhalteplatte 11 befestigt sein, an welcher die Düsen 2, 3 durch geeignete Mittel angebracht sind.

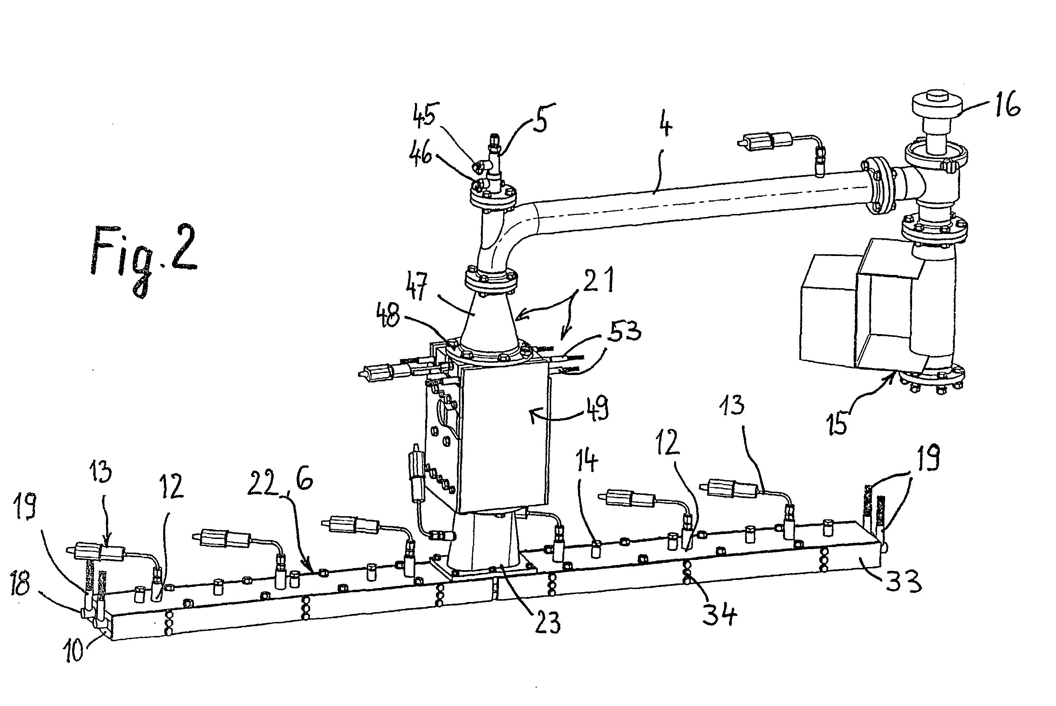

Gemäß Figur 1 sieht man, daß über die Länge des Rohrkörpers 6 verteilt Meßstellen 12 in Form von Temperatursensoren vorgesehen sind, deren Abgänge man bei 13 in den Figuren 2 und 3 nach oben geführt erkennt. Der Rohrkörper 6 wird sowohl auf seiner rechten als auch auf seiner linken Hälfte an drei Stellen über die Temperaturmeßstellen 12 überwacht, weshalb in Figur 1 bei der Aufteilung des Rohrkörpers 6 in "links" und "rechts" auf jeder Seite drei Temperaturen gemessen werden, nämlich Tr innen, Tr mitte und Tr außen. Das Gleiche gilt für "links", für welches "I" steht.

Für eine etwaige Druckmessung sind außerdem durch Endstopfen 14 verschlossene Öffnungen vorgesehen, durch die Druckmeßsensoren eingeführt werden können, hier aber nicht dargestellt sind. Unter anderem kann gemäß Figur 1 an der Verdampfungseinrichtung 21 im oberen Bereich die Temperatur der Heizung und im unteren Bereich die Temperatur der Abluft gemessen werden. Auch die Temperatur der sterilen Warmluft, der Zuluft, kann in der Zuführleitung 4 gemessen werden, wobei der in den Figuren 2 und 3 deutlicher gezeigte Durchflußmesser 15 nur schematisch angedeutet ist. Es versteht sich, daß durch Ventile 16 der Durchfluß der Warmluft regelbar ist.

Der Rohrkörper 6 mit dem von dem Längskanal 8 durchzogenen Grundkörper 7 weist auf den Außenseiten, die in Figur 6 links und rechts angedeutet sind und in Figur 4 oben und unten, nach außen offene Nuten 17 auf, in die bei der Herstellung von außen jeweils eine längliche Heizpatrone 18 einlegbar ist. Da es sich auf beiden Seiten des Längskanals 8 um je eine Nut 17 handelt, können also insgesamt zwei Heizpatronen 18 eingelegt werden. Gemäß den Figuren 2 und 3 stehen die beiden Heizpatronen 18 nach beiden Seiten hin (rechts und links) ein Stück weit heraus, wie man in der Draufsicht in Figur 6 für das rechte Ende vergrößert erkennt. Die Heizpatrone 18 ist im Querschnitt vergrößert in Figur 5 dargestellt mit ihrem elektrischen Anschluß 19 an einem Ende, zweckmäßig das aus dem Rohrkörper 6 herausstehende Ende. Die Heizpatrone 18 ist in ihrer Gesamtheit in drei Abschnitte 18a, 18b und 18c unterteilt, und jeder Abschnitt 18a, 18b, 18c ist mit einem eigenen elektrischen Anschluß 19 versehen, so daß man die jeweils drei Temperaturbereiche auf jeder Hälfte des länglichen Rohrkörpers 6 unterschiedlich ansteuern und erwärmen kann.

In die seitlich offenen Nuten 17 des Rohrkörpers 6 werden übrigens nach Einlegen der Heizpatronen 18 von außen her Druckstücke 32 eingesetzt und mittels Klemmplatten 33 mit Druckschrauben 34 festgeklemmt.

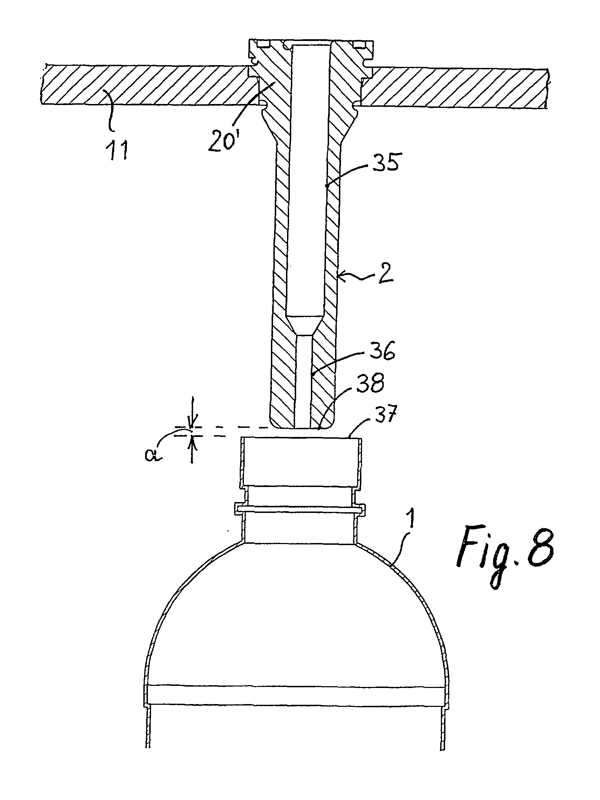

Eine erste Ausführungsform einer Düse, die mit 3 bezeichnet ist, ist in den Figuren 3 und 7 dargestellt. Von einem ringförmigen Befestigungsflansch 20, welcher oben die Verbindungsöffnung 9 hat, erstrecken sich ebene oder gekrümmte Außenwände 24 nach unten, so daß sich ein länglicher Raum für das Einströmen des konditionierten Gasgemisches von oben nach unten ergibt. Die Außenwand 24 ist unten mit einer Endkante 25 versehen, an welcher ein Sprühkanal 26 befestigt ist. Dieser hat die Form einer Rinne und ist ebenso wie der diese Rinne außen umgebende und durch die Außenwände 24 begrenzte Raum nach vorn und hinten in Blickrichtung auf die Figur 7 und entgegen dieser Richtung offen. In dieser Richtung senkrecht zur Papierebene der Figur 7 können bei dieser Ausführungsform als oben durch einen Dom verschlossene HDPE-Flaschen ausgestaltete Packungen intermittierend bewegt werden. Sie halten jeweils unter einer Düse bzw. in einem Düsenbereich an, in welchem der Sprühkanal 26 mit Sprühlöchern 27 versehen ist.

Durch den rinnenförmigen Sprühkanal 26 wird ein rinnenförmiger Innenraum 28 geschaffen, durch welchen der obere Teil der oben geschlossenen Packung 1 hindurchläuft, so daß durch die Sprühlöcher 27 in den Innenraum 28 eintretendes Gasgemisch auf die Oberflächen des oberen Teils der Packung 1 gelangt und diese Oberflächen sterilisiert. Nicht verwendetes oder verbrauchtes Abgas strömt dann in dem Innenraum in Blickrichtung der Figur 7 senkrecht zum Papier oder entgegen der Blickrichtung sowie unten in Richtung der gebogenen Pfeile 29 in die Umgebung ab. Dort kann das Abgas gesammelt werden. Im Falle einer nicht gezeigten Flaschenhalteplatte hängt die Packung 1 in nicht gezeigten Ausnehmungen derselben, so daß die Abgase über der Flaschenhalteplatte zur Seite weg strömen und dort aufgefangen werden können.

Zur Verbesserung des Rückstaues ist am oberen Ende im Bereich des Befestigungsflansches 20 neben der Verbindungsöffnung 9 eine Restriktorscheibe 30 in Form einer Blende mit einem Loch angebracht. Dieses Loch gibt eine Drosselöffnung 31 vor.

Eine alternative Ausführungsform einer mit 2 bezeichneten Düse ist in Figur 8 dargestellt. Sie hängt über einen Befestigungsflansch 20' in der Düsenhalteplatte 11 und ist dort befestigt. Die Haupterstreckung dieser länglichen Düse 2 liegt im wesentlichen vertikal, weshalb sich die Düse 2 von oben nach unten erstreckt und aus der Düsenhalteplatte 11 nach unten vorsteht. In etwa zwei Drittel bis drei Viertel der Gesamtlänge der Düse 2 ist diese von einem Durchflußkanal 35 größeren Durchmessers im Vergleich zu dem Ausströmungskanal 36 durchzogen. Beide Kanäle 35 und 36 befinden sich in Flucht zueinander. Der Ausströmungskanal 36 bildet für diese zweite Ausführungsform der Düse 2 die Drosselöffnung, so daß der Druckverlust des konditionierten Gasgemisches zwischen dem Inneren des Rohrkörpers 6 und der Umgebung hauptsächlich im Bereich des Ausströmungskanals 36 erzeugt wird. Diese in Figur 8 gezeigte zweite Ausführungsform der länglichen Düse 2 dient dem Einleiten des sterilisierenden Gasgemisches etwa vertikal nach unten in eine als PET-Flasche ausgestaltete Packung 1, die oben offen ist, und deren oberste Kante 37 in einem Abstand a von der Unterkante 38 der Düse 2 gehalten ist.

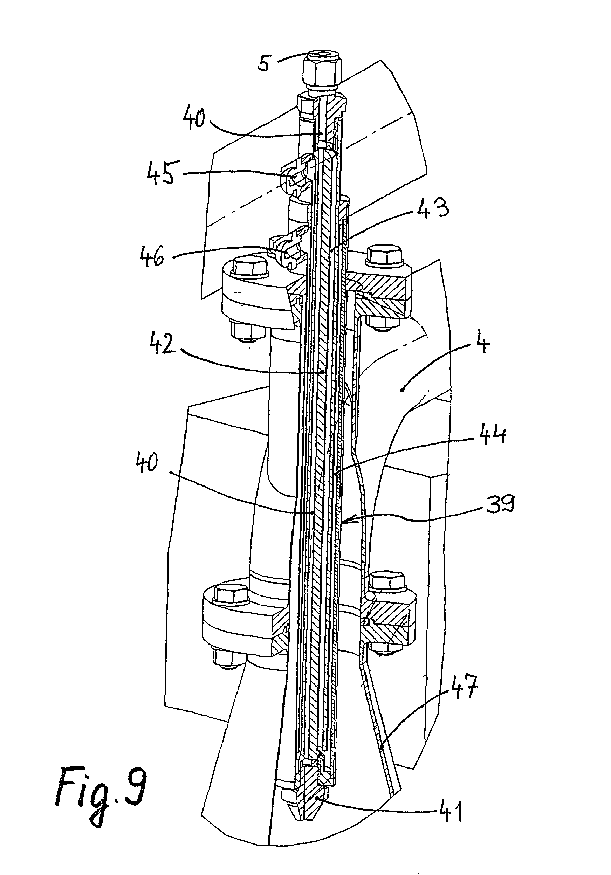

In Figur 9 erkennt man perspektivisch und teilweise in Querschnittsansicht die allgemein mit 39 bezeichnete Vernebelungsdüse, die an ihrem oberen Ende mit der Zuleitung 5 für flüssiges Wasserstoffperoxid verbunden ist, während der Raum um den unteren Bereich dieser Vernebelungsdüse 39 mit der Zuführleitung 4 für erwärmte Sterilluft in Verbindung steht. Man erkennt den im wesentlichen vertikal verlaufenden Fließkanal 40 für flüssiges Wasserstoffperoxid, welches bis zum Ein- und Austritt in die eigentliche Vernebelungsdüse, die hier als unteres Ende 41 bezeichnet ist, in flüssiger Phase bleibt. Hierfür ist der den Fließkanal 40 bildende Düsenkörper 42 außen von einer Doppelkammer 43, 44 umgeben, deren innerer Teil 43 neben dem unteren Ende 41 mit dem äußeren Teil 44 der Doppelkammer in Fließverbindung steht. Außerdem ist der innere Teil 43 der Doppelkammer oben mit einem Wasserzulauf 45 und daneben der äußere Teil 44 mit einem Wasserablauf 46 in Fließverbindung. Schließt man eine Kaltwasserleitung an den Wasserzulauf 45 an, dann kann der Düsenkörper 42 von dem einströmenden Kaltwasser gekühlt werden. Dieses Kaltwasser strömt vertikal entlang dem Düsenkörper 42 nach unten, tritt dort in den äußeren Teil 44 der Doppelkammer ein und strömt koaxial nach oben, um den äußeren Teil 44 der Doppelkammer durch den Wasserablauf 46 zu verlassen.

Das untere Ende 41 der Vernebelungsdüse 39 befindet sich etwa im mittleren Bereich einer kegelstumpfförmig dargestellten Verdampfungskammer 47. Der erweiterte Teil des Kegelstumpfes ist über einen Ringflansch 48 mit der aufstromigen Oberseite eines Erwärmungskörpers 49 verbunden. Dessen aufstromiger, warmer, ebener Oberfläche 50 wird Wasserstoffperoxid in Nebelform, vermischt mit der warmen Luft, im wesentlichen von oben nach unten zugeführt. Dadurch wird das mit dem Nebel versetzte Trägergas (warme, sterile Luft) schon an dieser Oberfläche 50 verdampft. Das verdampfte Gasgemisch strömt dann vertikal nach unten durch parallel zueinander verlaufende, den Erwärmungskörper 50 im wesentlichen vertikal und vollständig durchziehende Kanäle 51, deren warme Außenwände für eine weitere Erwärmung und gegebenenfalls Überhitzung des Gasgemisches sorgen. Längs des Erwärmungskörpers 49 ist ein Kranz von Heizstäben 52 eingesetzt, deren elektrische Versorgungsleitungen 53 radial oder zu einer oder mehreren Seiten (Figur 2) herausstehend gezeichnet sind.

### Bezugszeichenliste

- 1: Packung
- 2, 3: Düse
- 4, 5: Zuführleitung
- 6: Rohrkörper
- 7: Grundkörper
- 8: Längskanal
- 9: Verbindungsöffnung
- 10: Boden des Rohrkörpers 6
- 11: Düsenhalteplatte
- 12: Meßstelle
- 13: Abgang des Temperatursensors
- 14: Endstopfen
- 15: Durchflußmesser
- 16: Ventil
- 17: Nut
- 18: Heizpatrone
- 19: elektrischer Anschluß
- 20,20': Befestigungsflansche
- 21: Verdampfereinrichtung
- 22: Verteilerleitung
- 23: aufstromige Einspeisestelle
- 24: Außenwand
- 25: Endkante
- 26: Sprühkanal
- 27: Sprühloch
- 28: Innenraum
- 29: Fließrichtung des Abgases
- 30: Restriktorscheibe
- 31: Drosselöffnung
- 32: Druckstücke
- 33: Klemmplatte
- 34: Druckschraube
- 35: Durchflußkanal
- 36: Ausströmungskanal
- 37: oberste Kante der offenen Packung
- 38: Unterkante der Düse 2
- 39: Vernebelungsdüse
- 40: Fließkanal
- 41: unteres Ende
- 42: Düsenkörper
- 43: Doppelkammer, innerer Teil
- 44: Doppelkammer, äußerer Teil
- 45: Wasserzulauf
- 46: Wasserablauf
- 47: Verdampfungskammer
- 48: Ringflansch
- 49: Erwärmungskörper
- 50: warme Oberfläche
- 51: Kanal
- 52: Heizstab
- 53: elektrische Versorgungsleitung

- a: Abstand

## Patentansprüche

1. Vorrichtung zum gleichzeitigen Sterilisieren einer Mehrzahl von Packungen (1) mit Hilfe eines Wasserstoffperoxid und ein Trägergas enthaltenden Gasgemisches,
- mit Zuführleitungen für das Trägergas (4) und für Wasserstoffperoxid (5),
- einer Einrichtung (21) zum Verdampfen von Wasserstoffperoxid mit Wärme und zum Einmischen in das Trägergas,
- mit Zuführleitungen (4, 5) und einer im wesentlichen horizontal verlaufenden Verteilerleitung (6, 22) und
- mit über der jeweiligen Packung (1) angeordneten und mit der Verteilerleitung (6, 22) verbundenen Düsen (2, 3)
**dadurch gekennzeichnet, daß** die Verteilerleitung (6, 22) von der aufstromigen Stelle (23) der Einspeisung des konditionierten Gasgemisches bis zur Stelle (9) des Eintrittes vor die jeweilige Düse (2, 3) als einen Längskanal (8) enthaltender Rohrkörper (6) ausgestaltet ist mit mindestens einer sich über dessen Länge erstreckender, etwa rohrförmiger Heizpatrone (18) und verteilten Meßstellen (12) und daß die Heizpatrone (18) in wenigstens zwei Abschnitte (18a, 18b, 18c) aufgeteilt und durch Zufuhr elektrischer Energie gesteuert derart erwärmbar ist, daß sich die Temperatur (T) an den äußeren Enden des Rohrkörpers (6) von der Temperatur in der Mitte unterscheidet.

2. Vorrichtung nach Anspruch 1, **dadurch gekennzeichnet, daß** der Rohrkörper (6) die Form eines länglichen Steges hat mit einem massiven Grundkörper (7), der mittig von dem den Gaskanal (8) bildenden Längskanal (8) mit Verbindungsöffnungen (9) zu den Düsen (2, 3) durchzogen ist und der auf gegenüberliegenden Seiten des Gaskanales (8) und im Abstand zu diesem mit nach außen offenen Nuten (17) für das Einlegen einer Heizpatrone (18) versehen ist.

3. Vorrichtung nach Anspruch 1 oder 2, **dadurch gekennzeichnet, daß** jeder Abschnitt (18a, 18b, 18c) der Heizpatrone (18) unabhängig vom anderen beheizbar ist.

4. Vorrichtung nach einem der Ansprüche 1 bis 3, **dadurch gekennzeichnet, daß** die jeweilige Düse (2, 3), vorzugsweise über eine Düsenhalteplatte (11), mit den Verbindungsöffnungen (9) des Rohrkörpers (6) verbunden und an letzterem angebracht ist und eine Drosselöffnung (31, 36) hat.

5. Vorrichtung nach einem der Ansprüche 1 bis 4, **dadurch gekennzeichnet, daß** die Düse (3) einen die zu sterilisierenden Bereiche der Packung (1) wenigstens teilweise umgreifenden, durch einen auf gegenüberliegenden Seiten offenen, ringförmigen, mit Sprühlöchern (27) versehenen Sprühkanal (26) begrenzten Innenraum (28) und im Abstand vom Sprühkanal (26) angebrachte, ebenfalls seitlich teilweise offene Außenwände (24) aufweist (Figur 7).

6. Vorrichtung nach einem der Ansprüche 1 bis 5, **dadurch gekennzeichnet, daß** die Einrichtung zum Verdampfen von Wasserstoffperoxid mit Wärme und zum Einmischen in das Trägergas eine von einer Zuführleitung (5) für flüssiges Wasserstoffperoxid gespeiste Vernebelungsdüse (39) aufweist, die im mittleren Bereich einer Verdampfungskammer (47) aufstromig eines Erwärmungskörpers (49) angeordnet ist, zu dessen aufstromiger, warmer Oberfläche (50) Wasserstoffperoxid in Nebelform unter Vermischen mit warmem Trägergas zugeführt und durch Kanäle (51) in den Erwärmungskörper (50) derart geleitet wird, daß vor der Einspeisung in die Verteilerleitung (6, 22) eine Überhitzung des Gasgemisches erfolgt.

7. Vorrichtung nach einem der Ansprüche 1 bis 6, **dadurch gekennzeichnet, daß** wenigstens im abstromigen Endbereich des Erwärmungskörpers (49) einige Heizstäbe (52) eingesteckt sind.

8. Vorrichtung nach Anspruch 6, **dadurch gekennzeichnet, daß** die Zuführleitung (5) für flüssiges Wasserstoffperoxid durch ein Kühlfließmittel gekühlt ist.

## Claims

1. Apparatus for simultaneously sterilizing a multiplicity of packs (1) by means of a gas mixture containing hydrogen peroxide and a carrier gas, comprising
- supply lines for the carrier gas (4) and for hydrogen peroxide (5),
- a device (21) for evaporating hydrogen peroxide with heat and for mixing it into the carrier gas,
- supply lines (4, 5) and a substantially horizontally extending distributor line (6, 22), and
- nozzles (2, 3) which are arranged above the respective pack (1) and are connected to the distributor line (6, 22),
**characterized in that** the distributor line ( 6, 22) from the upstream location (23) of the infeed of the conditioned gas mixture to the location (9) of entry upstream of the respective nozzle (2, 3) is in the form of a tubular body (6) containing a longitudinal passage (8), with at least one substantially tubular heating cartridge (18) extending over the length of the body, and distributed measurement locations (12), and that the heating cartridge (18) is divided into at least two portions (18a, 18b, 18c) and can be heated controlledly by a supply of electrical energy in such a way that the temperature (T) at the outer ends of the tubular body (6) differs from that at the center.

2. Apparatus as set forth in claim 1 **characterized in that** the tubular body (6) is in the form of an elongate bar with a strong main body (7) through which there centrally passes the longitudinal passage (8) forming the gas passage (8) with communicating openings (9) to the nozzles (2, 3) and which is provided on opposite sides of the gas passage (8) and at a spacing with respect thereto with outwardly open grooves (17) for the insertion of a heating cartridge (18).

3. Apparatus as set forth in claim 1 or claim 2 **characterized in that** each portion (18a, 18b, 18c) of the heating cartridge (18) is heatable independently of the other.

4. Apparatus as set forth in one of claims 1 through 3 **characterized in that** the respective nozzle (2, 3) is connected preferably by way of a nozzle holding plate (11) to the communicating openings (9) of the tubular body (6) and is mounted to the latter and has a throttle opening (31, 36).

5. Apparatus as set forth in one of claims 1 through 4 **characterized in that** the nozzle (3) has an internal space (28) which at least partially embraces the regions of the pack (1) to be sterilized and is delimited by an annular spray passage (26) which is open on opposite sides and which is provided with spray holes (27) and outside walls (24) which are disposed at a spacing from the spray passage (26) and which are also laterally partially open (Figure 7).

6. Apparatus as set forth in one of claims 1 through 5 **characterized in that** the device for the evaporation of hydrogen peroxide with heat and for mixing it into the carrier gas has an atomization nozzle (39) which is fed by a supply line (5) for liquid hydrogen peroxide and which is arranged in the central region of a evaporation chamber (47) upstream of a heating body (49), to the upstream hot surface (50) of which hydrogen peroxide is fed in mist form with mixing with hot carrier gas and is passed through passages (51) into the heating body (50) in such a way that super-heating of the gas mixture occurs prior to being fed into the distributor line (6, 22).

7. Apparatus as set forth in one of claims 1 through 6 **characterized in that** some heating bars (52) are inserted at least in the downstream end region of the heating body (49).

8. Apparatus as set forth in claim 6 **characterized in that** the supply line (5) for liquid hydrogen peroxide is cooled by a cooling fluid.

## Revendications

1. Dispositif de stérilisation simultanée d'une pluralité d'emballages (1) à l'aide d'un mélange de gaz contenant du peroxyde d'hydrogène et un gazeux porteur, ledit dispositif comportant
- des conduites d'amenée du gaz porteur (4) et du peroxyde d'hydrogène (5),
- un dispositif (21) de vaporisation du peroxyde d'hydrogène à la chaleur et de mélange au gaz porteur,
- des conduites d'amenée (4, 5) et une conduite de distributeur (6, 22) s'étendant sensiblement horizontalement et
- des buses (2, 3) placées au-dessus des emballages respectifs (1 ) et reliées à la conduite de distributeur (6, 22),
**caractérisé en ce que** la conduite de distributeur (6, 22) est la forme, de l'emplacement aval (23) de l'alimentation en mélange de gaz conditionné jusqu'à l'emplacement (9) de la rentrée avant la buse respective (2, 3), d'un corps tubulaire (6) contenant un canal longitudinal (8) et comportant au moins une cartouche chauffante (18) sensiblement tubulaire s'étendant sur sa longueur et des emplacements de mesure répartis (12) et **en ce que** la cartouche chauffante (18) est divisée en au moins deux portions (18a, 18b, 18c) et peut être chauffée de façon commandée en amenant de l'énergie électrique de sorte que la température (T) aux extrémités extérieures du corps tubulaire (6) est différente de la température au milieu.

2. Dispositif selon la revendication 1, **caractérisé en ce que** le corps tubulaire (6) a la forme d'une nervure très allongée comportant un corps de base massif (7) qui s'étend au milieu du canal longitudinal (8) formant le canal de gaz (8) et doté d'ouvertures de liaison (9) jusqu'aux buses (2, 3), et qui est doté, du côté opposé du canal de gaz (8) et à distance de celui-ci, de gorges (17) ouvertes vers l'extérieur et destinées à l'insertion d'une cartouche chauffante (18).

3. Dispositif selon la revendication 1 ou 2, **caractérisé en ce que** chaque portion (18a, 18b, 18c) de la cartouche chauffante (18) peut être chauffée indépendamment des autres.

4. Dispositif selon l'une des revendications 1 à 3, **caractérisé en ce que** la buse respective (2, 3), est reliée, avantageusement par une plaque de support de buses (11), aux ouvertures de liaison (9) du corps tubulaire (6) et est montée sur ce dernier et comporte une ouverture d'étranglement (31, 36).

5. Dispositif selon l'une des revendications 1 à 4, **caractérisé en ce que** la buse (3) comporte un espace intérieur (28) qui enferme au moins partiellement des zones à stériliser de l'emballage (1) et qui est délimité par un canal d'atomisation (26) annulaire ouvert du côté opposé et doté de trous d'atomisation (27), et comporte des parois extérieures (24) également partiellement ouvertes latéralement et montées à distance du canal d'atomisation (26) (Figure 7).

6. Dispositif selon l'une des revendications 1 à 5, **caractérisé en ce que** le dispositif de vaporisation de peroxyde d'hydrogène à la chaleur et de mélange au gaz porteur comporte une buse d'atomisation (39) qui est alimentée en peroxyde d'hydrogène liquide par une conduite d'amenée (5) et qui est agencée dans la région médiane d'une chambre de vaporisation (47) en amont d'un corps chauffant (49) dont la surface chaude amont (50) reçoit du peroxyde d'hydrogène qui se présente sous forme atomisée en étant mélangé à un gaz porteur chaud et qui est introduit par des canaux (51) dans le corps chauffant (50) de sorte qu'une surchauffe du mélange de gaz est effectuée avant l'introduction dans la conduite de distributeur (6, 22).

7. Dispositif selon l'une des revendications 1 à 6, **caractérisé en ce qu'**un certain nombre de barres chauffantes (52) sont insérées au moins dans la région d'extrémité aval du corps chauffant (49).

8. Dispositif selon la revendication 6, **caractérisée en ce que** la conduite d'amenée (5) destinée au peroxyde d'hydrogène liquide est refroidie par un fluide de refroidissement.
